# EUROPEAN PATENT APPLICATION

(11) **EP 3 809 660 A1**
(43) Date of publication of application: **21.04.2021**
(21) Application number: 19203644.0
(22) Date of filing: 16.10.2019
(51) Int. Cl.: H04L 29/06, G06F 21/30, G06F 21/60, G06F 21/64, G16H 40/60

(54) **METHOD FOR OPERATING A MEDICAL SYSTEM, MEDICAL SYSTEM, AND SECURITY MODULE**

(71) Applicant: Roche Diabetes Care GmbH, 68305 Mannheim (DE); F. Hoffmann-La Roche AG, 4070 Basel (CH)
(72) Inventor: Kreuzer, Andreas, 68305 Mannheim (DE)
(74) Representative: Bittner, Thomas L.

(57) **Abstract**

A method for operating a medical system is disclosed (1) having a remote control device (2), a security module (3), and a medical device (4). The method comprises providing a pair of keys for asymmetric cryptography, the pair of keys comprising a public key and a private key, wherein the public key is provided in both the remote control device (2) and the medical device (4), and the private key is provided in the security module (3); in the remote control device (2), generating an encrypted control command encrypted by applying the public key to a control command configured to control operation of the medical device (4); receiving the encrypted control command in the security module (3); in the security module (3), decrypting the encrypted control command by applying the private key, the decrypting comprises determining that the control command was encrypted by applying the public key; in the security module (3), generating a security module encrypted control command by applying the private key to the decrypted control command or an amended medical control command derived from the decrypted control command and configured to control operation of the medical device (4); receiving the security module (3) encrypted control command in the medical device (4); in the medical device (4), decrypting the security module encrypted control command by applying the public key; and controlling operation of the medical device (4) according to one of the control command and the amended control command, if the control command or the amended control command has been confirmed by a user confirmation input received in one of the security module (3), and the medical device (4). Further, a medical system (1) and a security module are disclosed. (Fig. 1)

## Description

The present disclosure refers to a method for operating a medical system. The disclosure also refers to a medical system, and a security module.

### Background

Such medical system may be provided with a remote control device configured to provide control commands for operating a medical device.

Document EP 3 155 958 A2 discloses a method for operating a system, the system comprising a medical device, having at least one of a sensor device for sensing medical data and a medication delivery device for delivering medication, a portable electronic consumer device, an intermediate device provided with a first communication protocol for data communication with the portable electronic consumer device and a second communication device, and a control module provided in the intermediate device. The method comprises, in the control module, receiving control data from the portable electronic consumer device by a receiving functionality provided in the control module, the control data being configured for controlling operation of the medical device, determining whether the control data can be confirmed by a confirmation functionality provided in the control module, and, if the control data are confirmed, transmitting the control data to the medical device by a transmission functionality provided in the control module.

### Summary

It is an object of the present disclosure to provide a method for operating a medical system for improving reliable and secure operation of a medical device provided in the medical system. It is a further object of the present disclosure to provide a medical system and/or a security module for improving reliable and secure operation thereof in a medical system

For solving at least one of the objects, a method for operating a medical system according to claim 1 is provided. Further, a medical system, and a security module according to claims 11 and 13, respectively, are provided. Further embodiments are referred to in dependent claims.

According to one aspect, a method for operating a medical system having a remote control device, a security module, and a medical device is provided. The method comprises: providing a pair of keys for asymmetric cryptography, the pair of keys comprising a public key and a private key, wherein the public key is provided in both the remote control device and the medical device, and the private key is provided in the security module; in the remote control device, generating an encrypted control command encrypted by applying the public key to a control command configured to control operation of the medical device; receiving the encrypted control command in the security module; in the security module, decrypting the encrypted control command by applying the private key, the decrypting comprises determining that the control command was encrypted by applying the public key; in the security module, generating a security module encrypted control command by applying the private key to the decrypted control command or an amended medical control command derived from the decrypted control command and configured to control operation of the medical device; receiving the security module encrypted control command in the medical device; in the medical device, decrypting the security module encrypted control command by applying the public key; and controlling operation of the medical device according to one of the control command and the amended control command, if the control command or the amended control command has been confirmed by a user confirmation input received in one of the security module, and the medical device.

According to another aspect, a medical system is provided, the system comprising a remote control device, a security module; and a medical device. The system components are configured to: provide a pair of keys for asymmetric cryptography, the pair of keys comprising a public key and a private key; provide the public key in both the remote control device and the medical device; provide the private key in the security module; in the remote control device, generate an encrypted control command encrypted by applying the public key to a control command; receive the encrypted control command in the security module; in the security module, decrypt the encrypted control command by applying the private key, the decrypting comprises determining whether the control command was encrypted by applying the public key; in the security module, generate a security module encrypted control command by applying the private key to the decrypted control command or an amended medical control command derived from the decrypted control command and configured to control operation of the medical device; receive the security module encrypted control command in the medical device; in the medical device, decrypt the security module encrypted control command by applying the public key; and control operation of the medical device according to one of the control command and the amended control command, if the control command or the amended control command has been confirmed by a user confirmation input received in one of the security module, and the medical device.

According to still another aspect, a security module for a medical system is provided. The security module comprises: a data memory comprising a private key assigned to a pair of keys for asymmetric cryptography, the pair of keys comprising the private key and a public key; one or more data processors, and a data communication interface for data communication with a remote control device and a medical device. The one or more data processors are configured to: receive, from the remote control device, an encrypted control command encrypted by applying the public key to a control command configured to control operation of the medical device; decrypt the encrypted control command by applying the private key, the decrypting comprises determining whether the control command was encrypted by applying the public key; generate a security module encrypted control command by applying the private key to the decrypted control command or an amended medical control command derived from the decrypted control command and configured to control operation of the medical device; transmit the security module encrypted control command to the medical device.

The technology proposed provides for improved security of data communication within the medical system between the remote control device and the medical device.

The remote control device may be a non-regulated device. In such case as well the technology proposed ensures save data communication and operation of the medical device. With respect to non-regulated device, for example, the U.S. food and drug administration (FDA) regulates devices to be applied with or used as a medical device to ensure their safety and effectiveness. For example, there is a national program designed to control unnecessary exposure, and ensure a safe and efficient use of ionizing and non-ionizing radiation-emitting electronic products. Regulations, for example by the FDA, provide provisions, that is, regulatory requirements, that define general level of control over these products. A non-regulated device within the meaning of the present disclosure is a device which may or may not fulfil the provisions of such regulations, but has not been certified to fulfil such regulations. In other countries similar official provisions apply. The remote control device among other may be a Smartphone, a Personal Digital Assistant, a handheld computer, a notebook, or a tablet.

The security module may be a separate device, i.e. separate from the remote control device and the medical device. Alternatively, the security module may be integrated in or attached to either the remote control device or the medical device. Further alternatively, the remote control device and the medical device may each comprise an integrated or attached security module.

In the method for operating the medical system, the security module may prevent the control command from transmission to the medical device if it is concluded or determined by the security module that the control command has not been correctly encrypted by the public key. Also, transmission of the control command may be prevented if it is determined by the security module that the encrypted control command received has not been submitted by the remote control device. In response, the security module may delete the encrypted control command and / or the decrypted control command, thereby, preventing storage of such data in the security module.

The security module encrypted control command may be transmitted to the medical device directly from the security module to the medical device. In an alternative embodiment, the security module encrypted control command is transmitted indirectly from the security module via the remote control device and / or some intermediate device(s) to the medical device.

The pair of keys for asymmetric cryptography may be stored in the security module at the time of manufacturing the security module. In case of providing the pair of keys after manufacturing in the security module, it may still be done before the security module is brought into the field (e.g. delivered to a customer or user).

The pair of keys provides the option for encrypted and protected data communication between the security module and the remote control device as well as between the medical device and the remote control device. The remote control device itself is not in possession (free) of the private key. The same applies to the medical device. But, the security module may be provided with both the private key and the public key as well. The security module may provide the public key to the public, so that the security module may communicate with a medical device and/or a remote control, which initially do not have been provided with the public key, but which receive the public key from the security module.

The components of the medical system may be provided with one or more data communication protocols for data communication. There may be a first data communication protocol applied for data communication between the remote control device and the security module. A second data communication protocol may be provided for data communication between the security module and the medical device. In an alternative embodiment, the same data communication protocol may be applied for both data communication between the remote control device and the security module and data communication between the medical device and the security module.

The components of the medical system are provided with functionality for performing or implementing the steps or measures of the technology proposed, such as encryption functionality and decryption functionality, by one or more software applications running on the respective system component.

The remote control device may be provided with a software application for generating and encrypting control commands configured to control operation of the medical device. Such control command may also be referred to as medical device control command. Before using such software application on the remote control device, the user may be required to "successfully pass" at two factor authentication known as such in different ways. Similarly, a two factor authentication may be applied before the user is allowed to use the security module and / or the medical device.

In the method, the generating of the security module encrypted medical device may further comprise the following: applying a command security check for the encrypted control command, comprising determining whether the control command is an approved control command approved for remote control of the medical device according to approved command control information provided in the security module; and generating the security module encrypted control command, if the control command is determined an approved control command. In the command security check the security module will determine whether the control command received is allowed for the medical device. It may be checked whether the remote control device from which the control command has been received is allowed for sending or applying the control command to the medical device. The approved command control information (data) provided in the security module may be identifying one or more control commands allowed to be applied to the medical device and / or control commands allowed for the remote control device only. Also, control command(s) not allowed for application by the remote control device may be indicated by the approved command control information. The command security check may comprise determining whether control data (control signals) provided or defined by the control command are within approved limits such as a limit for a pump volume to be delivered or applied by the medical device comprising a pump. For example, it may be checked whether delivery of an amount of insulin defined by the control command is within amount limits.

The generating of the security module encrypted control command may further comprise: applying an error-detecting check for the encrypted control command, comprising determining whether the control command was correctly encrypted by the remote control device; and generating the security module encrypted control command, if the control command was correctly encrypted. The error-detecting check may comprise a cyclic redundancy check (CRC) which is an error-detecting code used in digital networks or storage devices to detect accidental changes to data. The CRC check is known as such and may be applied for further improving security of data communication in the medical system.

In the method, the generating of the security module encrypted control command my further comprise: applying a device security check for the encrypted control command, comprising determining whether the control command is received from an approved remote control device approved for remote control of the medical device according to approved device control information provided in the security module; and generating the security module encrypted control command, if the remote control device is determined to be an approved remote control device. The device security check may be applied in the security module based on the approved device control information which is identifying one or more remote control devices allowed or approved for remotely controlling operation of the medical device. The approved device control information may be stored in a local storage or memory of the security module. Alternatively, the security module may receive such information through wireless data communication from a remote server device.

The method may further comprise, in the medical device, applying for the security module encrypted control command at least one of the command security check, the error-detecting check, and the device security check. The different checks described for application with the encrypted control command in the security module may be applied to the security module encrypted control command in the medical device itself for further improving security with respect to data communication and controlling of operation of the medical device. For example, the device security check may be configured to determine whether the security module from which the security module encrypted control command has been received is approved for submitting such command to the medical device.

The providing of the pair of keys for asymmetric cryptography may comprise providing the private key in a read only data memory in the security module. Storage devices characterized as "read only memory" are available in different types as such. The read only data memory, for example, may be implemented by a physical unclonable function (PUF) which is a physically-defined "digital fingerprint" that serves as a unique identity for a semiconductor device such as microprocessor. A PUF is a physical entity embodied in a physical structure. PUFs are usually implemented in integrated circuits. The private key for asymmetric cryptography may be stored in an dedicated memory allocation. The private key and/or the public key for asymmetric cryptography may be stored in a EEPROM, i.e. electrically erasable programmable read-only memory.

The method may further comprise providing the security module as a device component in one of the remote control device and the medical device. The security module may be implemented with the remote control device or the medical device. For example, the security module may be a plug-in component of one of such devices. One or more software applications may be running on the remote control device or the medical device for implementing functionality provided with the security module.

The method may further comprise providing the security module as a portable device separated from the remote control device, and the medical device. In such embodiment, the security module is provided by a portable device which is separated from both the remote control device and the medical device. The portable device is configured to have wireless data communication with the remote control device and the medical device applying one or more data communication protocols.

In the method, the confirming of the device control command or the amended control command may comprise the following: outputting command user information through an output device of a user interface provided on at least one of the remote control device, the security module, and the medical device, the command user information being indicative of the device control command or the amended device control command; and receiving the user confirmation input through an input device of the user interface. The user interface allows for outputting information or data such as user information or data, and receiving user input. It may be implemented by one or more software and hardware components. The user interface of the medical system may be provided with separate user interfaces provided with the system components such as the remote control device, the security module, and the medical device. The command user information may be provided by audio and / or video data outputted by the output device. The output device may, for example, comprise the signaling device such as one or more (colored) lamps. Different types of input devices may be used such as switch, button, touch sensitive device, and / or voice recording for receiving user input in one or more of the system components of the medical system. The respective system component comprising a user interface may be further configured to transmit a user confirmation input received via the user interface to the security module and/or the medical device for further processing.

In the method, the controlling of the operation may further comprise controlling operation of a medical device selected from the following group: medical pump device, insulin pump, and blood sugar measurement device.

The remote control device may be a portable control device. The control device may be portable. For example, a consumer electronic device may implement the remote control device such as mobile phone, a tablet computer, and a laptop.

The embodiments described for the method above may apply to the medical system and / or the security module *mutatis mutandis.*

### Description of further embodiments

Following, further embodiments are described by referring to figures. In the figures show:
Fig. 1 a schematic representation of a medical system comprising a remote control device, a security module, and a medical device; and
Fig. 2 a schematic flow diagram for a method of operating the medical system.

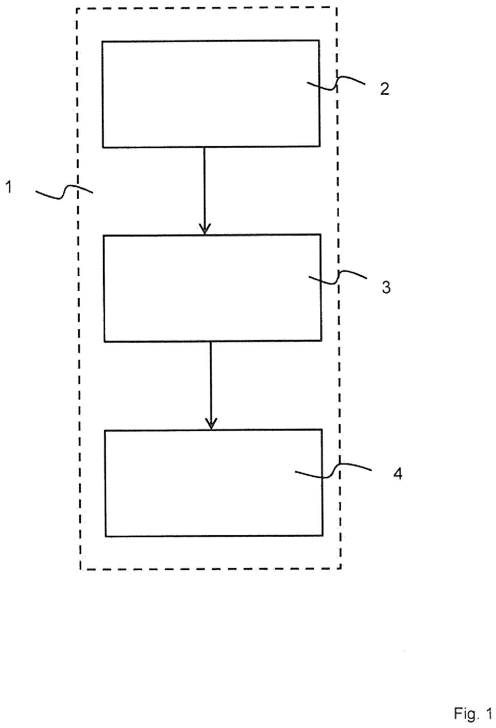

Fig. 1 shows a schematic representation of a medical system 1 comprising the following components: a remote control device 2, a security module or device 3, and a medical device 4. The medical device 4, for example, may be provided with a pump for delivering medication, e.g. insulin pump, a measurement device such as a sensor device for gathering or collecting medical data, or an analysis device such as a device for determining a sample of a bodily fluid. Such components of the medical system 1 are provided with one or more data communication protocols for wireless and / or wired data communication. Different technologies available as such may be applied for implementing data communication between the components of the medical system 1 such as Bluetooth or nearfield communication (NFC). Different software applications can be implemented on the components of the medical system 1 for providing different functionalities such as encryption and decryption of data or information. Other functionalities refer to data transmission, data reception, data storage, and data processing. Each of the components of the medical system 1 may comprise one or more data processors configured to process electronic data. Also, the components may have one or more storage devices for storing electronic data locally. In addition, data may be received from and / or transmitted to one or more remote server devices.

The remote control device 2 may be provided as a consumer electronic device such as mobile phone, tablet computer, or laptop. A software application is running on the remote control device 2 for at least generating control commands (also referred to as device control commands or medical device control commands) configured to control operation of the medical device 4. A control command is configured to control at least one functionality provided for the medical device 4 in a mode of operation. The remote control device 2 may be configured to apply a two factor authentication before a user is allowed to use the software application providing functionality for generating control commands.

In the embodiment shown in Fig. 1, the components of the medical system 1 are shown to be separated system components. In an alternative embodiment, the security module 3 may be part of one of the remote control device 2 and the medical device 4, for example, a plug-in device component.

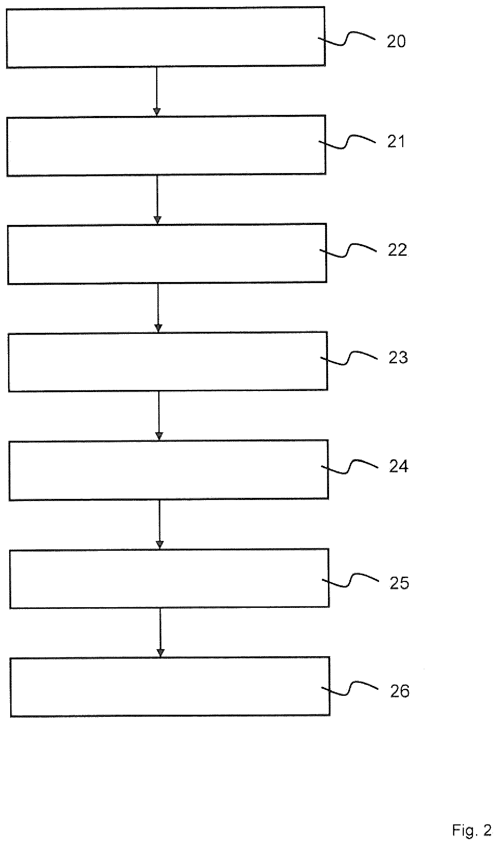

Referring to Fig. 2, a method for operating the medical system 1 provided with the remote control device 2, the security module 3, and the medical device 4 is described. In step 20, a pair of keys for asymmetric cryptography is provided in the medical system 1. The pair of keys comprises a public key and a private key. A cryptography infrastructure of the medical system 1 may comprise one or more further pairs of keys for asymmetric cryptography. The public key of the pair of keys is provided at least to both the remote control device 2 and the medical 4. A copy of the public key may also be available in the security module 2. The private key is provided in the security module 3. In conclusion, neither the remote control device 2 nor the medical device 4 have received or have access to the private key which is kept to the security module 3 in the embodiment described. The pair of keys is assigned to security module 2 in the medical system 1.

If a user of the mode control device 2 wants to apply controlling of operation to the medical device 4, in response to a user input received in the remote control device 2, a control command is generated by the software application running on the remote control device 2 in step 21. The control command is configured to control operation of the medical device 4. For example, the control command may be provided for controlling application of medication by the medical device 4. For example, if the medical device 4 is provided with a pump device for delivering medication such as insulin, a pump control command is generated, e.g. an amount of medication is to be delivered by the pump device.

Following, in step 22 an encrypted control command is generated in the remote control device 2 by applying the public key to the control command.

The encrypted control command is transmitted from the remote control device 2 to the security module 3 in step 23. In step 24, the security module 3 is decrypting the encrypted control command by applying the private key. In doing so, it is determined by the security module 3 whether the control command was correctly encrypted by applying the public key. Further, the security module 3 may check whether the encrypted control command has been received from the remote control device 2. The security module 3 may confirm that the remote control device 2 is approved for providing control commands for the medical device 4. Information about approved remote control devices may be provided in a storage device of the security module 3. Alternatively or in addition, such information may be received from a remote server device. The security module 3 may also apply an error-detecting check for the encrypted control command received from the remote control device 2. For example, a cyclic redundancy test (CRC) check may be performed. One or more security checks may be applied by the security module 3.

If it is confirmed in the security module 3 that the received control command is determined to be correct (allowed) by one or more of the security checks, the security module 3 will generate a security module encrypted control command by applying the private key to the control command or an amended control command derived from the control command in step 24. For example, the amended control command may limit one or more operation parameters to limits allowed, such operation parameter(s) indicating limits in the control command not allowed for the medical device 4. The security module encrypted control command is transmitted from the security module 3 to the medical device 4 in step 25. In response, the medical device 4 will decrypt the security module encrypted control command by applying the public key.

In step 26, the operation of the medical device 4 is controlled according to one of the control command and the amended control command. Such controlling of operation is applied, if the control command or the amended control command has been confirmed by a user confirmation. Such user confirmation is received in response to outputting command user information through an output device provided with a user interface of the medical system 1, the command user information being indicative of the device control command or the amended device control command. By such command user information the user is informed about the control command / amended control command intended to be applied to the medical device 4. The user can check whether such control command shall be applied or not. By inputting the user confirmation input through an input device of the user interface of the medical system 1 the user is confirming that the control command can be applied.

The command user information may be provided with audio / or video data. The command user information may be outputted through an output device of one of the remote control device 2, the security module 3, and the medical device 4. Audio data and / or video data may be outputted for providing the command user information to the user. For example, the command user information may indicate to the user a functionality of the medical device 4 which is to be controlled by the control command. Also, the command user information may indicate one or more control parameter, according to the control command, to be applied for operating the medical device 4. For the operation parameters parameter limits may be indicated by the command user information.

The command user information output may be generated in the security module 3 in response to receiving the encrypted control command. As an alternative or in addition, command user information may be generated and outputted by the medical device 4 after receiving the security module encrypted control command. Thus, controlling operation of the medical device 4 according to the control command or the amended control command will only be applied after receiving user input confirmation.

It may be provided that only the remote control device 2 comprise functionality for generating the control command, but not the security module 3. On the other hand, only the security module 3 is provided with the private key. Thus, only the security module 3 (different from the remote control device 2) is enabled to generate the security module encrypted control command which can be decrypted in the medical device 4 for actual control of operation of the medical device 4. There is no need and no requirement for the security module 3 being provided with functionality for generating control commands, since such control commands are received from the remote control device 2. Thus, the security module 3 is provided free of functionality for generating control commands.

## Claims

1. Method for operating a medical system (1) having a remote control device (2), a security module (3), and a medical device (4), the method comprising:
- providing a pair of keys for asymmetric cryptography, the pair of keys comprising a public key and a private key, wherein the public key is provided in both the remote control device (2) and the medical device (4), and the private key is provided in the security module (3);
- in the remote control device (2), generating an encrypted control command encrypted by applying the public key to a control command configured to control operation of the medical device (4);
- receiving the encrypted control command in the security module (3);
- in the security module (3), decrypting the encrypted control command by applying the private key, the decrypting comprises determining that the control command was encrypted by applying the public key;
- in the security module (3), generating a security module encrypted control command by applying the private key to the decrypted control command or an amended medical control command derived from the decrypted control command and configured to control operation of the medical device (4);
- receiving the security module (3) encrypted control command in the medical device (4);
- in the medical device (4), decrypting the security module encrypted control command by applying the public key; and
- controlling operation of the medical device (4) according to one of the control command and the amended control command, if the control command or the amended control command has been confirmed by a user confirmation input received in one of the security module (3), and the medical device (4).

2. Method of claim 1, wherein the generating of the security module encrypted control command further comprises:
- applying a command security check for the encrypted control command, comprising determining whether the control command is a control command approved for remote control of the medical device (4) according to approved command control information provided in the security module (3); and
- generating the security module encrypted control command, if the control command is determined an approved control command.

3. Method of claim 1 or 2, wherein the generating of the security module encrypted control command further comprises:
- applying an error-detecting check for the encrypted control command, comprising determining whether the control command was correctly encrypted by the remote control device (2); and
- generating the security module encrypted control command, if the control command was correctly encrypted.

4. Method of at least one of the preceding claims, wherein the generating of the security module encrypted control command further comprises:
- applying a device security check for the encrypted control command, comprising determining whether the control command is received from a remote control device approved for remote control of the medical device (4) according to approved device control information provided in the security module (3); and
- generating the security module encrypted control command, if the remote control device is determined to be an approved remote control device.

5. Method of at least one of the claims 2 to 4, further comprising, in the medical device (4), applying for the security module encrypted control command at least one of the command security check, the error-detecting check, and the device security check.

6. Method of at least one of the preceding claims, wherein the providing of the pair of keys for asymmetric cryptography comprises providing the private key in a read only data memory in the security module (3).

7. Method of at least one of the preceding claims, further comprising providing the security module (3) as a device component in one of the remote control device (2), and the medical device (4).

8. Method of at least one of the claims 1 to 6, further comprising providing the security module (3) as a portable device separated from the remote control device (2), and the medical device (4).

9. Method of at least one of the preceding claims, wherein the confirming of the device control command or the amended control command comprises
- outputting command user information through an output device of a user interface provided on at least one of the remote control device (2), the security module (3), and the medical device (4), the command user information being indicative of the device control command or the amended device control command; and
- receiving the user confirmation input through an input device of the user interface.

10. Method of at least one of the preceding claims, wherein the controlling of the operation further comprises controlling operation of a medical device (4) selected from the following group: medical pump device, insulin pump, and blood sugar measurement device.

11. A medical system (1), comprising the following system components
- a remote control device (2);
- a security module (3); and
- a medical device (4);
the system components configured to
- provide a pair of keys for asymmetric cryptography, the pair of keys comprising a public key and a private key;
- provide the public key in both the remote control device (2) and the medical device (4);
- provide the private key in the security module (3);
- in the remote control device (2), generate an encrypted control command encrypted by applying the public key to a control command;
- receive the encrypted control command in the security module (3);
- in the security module (3), decrypt the encrypted control command by applying the private key, the decrypting comprises determining whether the control command was encrypted by applying the public key;
- in the security module (3), generate a security module encrypted control command by applying the private key to the decrypted control command or an amended medical control command derived from the decrypted control command and configured to control operation of the medical device (4);
- receive the security module (3) encrypted control command in the medical device (4);
- in the medical device (4), decrypt the security module encrypted control command by applying the public key; and
- control operation of the medical device (4) according to one of the control command and the amended control command, if the control command or the amended control command has been confirmed by a user confirmation input received in one of the security module (3), and the medical device (4).

12. Medical system (1) of claim 11, wherein the remote control device (2) is a portable control device.

13. A security module for a medical system (1), comprising
- a data memory comprising a private key assigned to a pair of keys for asymmetric cryptography, the pair of keys comprising the private key and a public key;
- one or more data processors; and
- a data communication interface for data communication with a remote control device (2) and a medical device (4);
wherein the one or more data processors are configured to
- receive, from the remote control device (2), an encrypted control command encrypted by applying the public key to a control command configured to control operation of the medical device (4);
- decrypt the encrypted control command by applying the private key, the decrypting comprises determining whether the control command was encrypted by applying the public key;
- generate a security module encrypted control command by applying the private key to the decrypted control command or an amended medical control command derived from the decrypted control command and configured to control operation of the medical device (4);
- transmit the security module encrypted control command to the medical device (4).
